Europäisches Patentamt

European Patent Office

Office européen des brevets

(11) Publication number: **0 574 086 A2**

## EUROPEAN PATENT APPLICATION

(21) Application number: 93201619.9

(22) Date of filing: 07.06.93

(51) Int. Cl.⁵: **C11D 3/34**, C11D 1/14, C11D 1/37

(30) Priority: 08.06.92 US 894882

(43) Date of publication of application:
15.12.93 Bulletin 93/50

(84) Designated Contracting States:
AT BE CH DE DK ES FR GB IE IT LI LU NL SE

(71) Applicant: **Colgate-Palmolive Company**
**300 Park Avenue**
**New York, N.Y. 10022-7499(US)**

(72) Inventor: **Marschner, Frank W.**
**3 Old Farm Road**
**Whitehouse Station, New Jersey(US)**

(74) Representative: **Smulders, Theodorus A.H.J.,**
**Ir. et al**
**Vereenigde Octrooibureaux**
**Nieuwe Parklaan 97**
**NL-2587 BN 's-Gravenhage (NL)**

(54) Alkyl sulfates as viscosity modifiers in anionic surfactant compositions.

(57) A method for adjusting the viscosity of anionic surfactant compositions is provided by combining anionic surfactant compositions with a viscosity modifier comprising alkyl sulfates of the formula:

$R\text{-}OSO_3M$

in which R is a straight or branched chain alkyl of from 7 to 10 carbons and M is an alkali metal. The viscosity modifier increases the salt tolerance levels of the anionic compositions without reducing peak viscosity.

EP 0 574 086 A2

Field of Invention

This invention relates to the use of alkyl sulfates as viscosity modifiers in anionic surfactant compositions. More particularly, it concerns a method for adjusting the viscosity of anionic surfactant compositions using alkyl sulfates of the formula:

$$R\text{-}OSO_3M$$

in which R is a straight or branched chain alkyl of from 7 to 10 carbons and M is an alkali metal to increase the salt tolerance level of the compositions without reducing peak viscosity.

Background Art

Generally, most anionic surfactant products, including shampoos, are customarily thickened by the addition of salt. Sodium chloride is typically used but sodium sulfate and ammonium chloride or sulfate are also used. A commonly used anionic surfactant system incorporated into shampoo compositions is sodium laureth (2EO) sulfate (SLES). Compositions containing SLES at low to moderate active ingredient (AI) levels generally do not present a problem of extremely high viscosities. However, with recent incorporation of ammonium lauryl sulfate (ALS) in shampoo compositions at moderate to high AI levels, the problem of very high or irreversibly low viscosities occurs. Problems of this kind are directly related to salt tolerance levels of the anionic surfactants used in the compositions as shown in their viscosity/salt curves.

The prior art has disclosed use of certain sulfates or sulfonate additives as viscosity regulators for particular anionic surfactant compositions. U.S. Patent No. 4,675,128 to Linde et al. is concerned with the use of alkane sulfonates as a viscosity regulator for anionic surfactant compositions comprised of an alpha-sulfofatty acid ester salt. The alkane sulfonates of Linde et al. have an average of from 11 to 21 carbon atoms.

Also, Great Britain Patent 1,437,089 to Albright and Wilson discloses use of certain sulfates or sulfonate additives as viscosity modifiers of aqueous anionic compositions comprising from about 40 to 75% of an alkyl ether sulfate. A preferred class of viscosity modifiers includes alkyl sulfates in which the alkyl group contains 1 to 6 carbon atoms.

Both U.S. Patent Nos. 3,899,488 to Messenger et al. and 3,893,955 to Hewitt et al. disclose viscosity modifiers for aqueous concentrates of alkyl ether sulfates. However, the viscosity modifier in Messenger et al. employs various aromatic sulphonates and Hewitt et al. uses water soluble salts of various carboxylic acids.

The present invention is directed to the use of alkyl sulfates, preferably sodium n-decyl sulfate, to adjust the viscosity of anionic surfactant compositions containing ammonium or sodium alkyl sulfates or alkyl ether sulfates. In particular it concerns a method for adjusting the viscosity of anionic surfactant compostions using alkyl sulfates to increase the salt tolerance levels of the compositions without reducing peak viscosity. It will be appreciated that advantage over known methods is obtained by providing a method which increases the salt tolerance levels of anionic compositions without reducing peak viscosity.

The invention further illustrates the limitations and potential problems using commonly known thinning agents and demonstrates how the viscosity of anionic surfactant compositions containing ammonium lauryl sulfates can be adjusted by the addition of alkyl sulfates, particularly, sodium n-decyl sulfate either alone or in conjunction with a thinning agent such as propylene glycol. In addition, the invention method extends to other anionic systems such as sodium lauryl sulfate and sodium laureth (2EO) sulfate.

Accordingly, it is a broad object of the invention to provide a viscosity modifier of anionic surfactant compositions which comprises an alkyl sulfate of the formula $R\text{-}OSO_3M$ in which R is a straight or branched chain alkyl of from 7 to 10 carbons and M is an alkali metal.

A more specific object of the invention is to provide a viscosity modifier which increases the salt tolerance levels of anionic surfactant compositions.

Another object of the invention is to provide a method for adjusting the viscosity of anionic surfactant compositions to produce compositions with higher salt tolerance levels.

A further specific object of the invention is to provide a method for adjusting the viscosity of anionic surfactant compositions using sodium n decyl sulfate to increase the salt tolerance levels of the compositions without reducing peak viscosity.

Disclosure of the Invention

In the present invention, these purposes, as well as others which will be apparent, are achieved generally by providing a viscosity modifier of anionic surfactant compositions using alkyl sulfates of the formula:

$R-OSO_3M$

in which R is a straight or branched chain alkyl of from 7 to 10 carbons and M is an alkali metal. Sodium n-decyl sulfate is the preferred alkyl sulfate used in the invention. The viscosity modifier is used to increase the salt tolerance level of the anionic compositions without reducing peak viscosity. Preferably the anionic surfactant compositions comprise ammonium or sodium alkyl sulfates or alkyl ether sulfates.

The method for adjusting the viscosity of anionic surfactant compositions is accomplished by combining with the viscosity modifier to form a composition having a higher salt tolerance level. The viscosity modifier is present in the range of 0.05 to 5% of the composition and the anionic surfactant is present in the range of 8 to 25% of the composition.

An agent may be added to the resultant composition to adjust the composition to a desired viscosity. The agent may be a thickening agent comprised of salt or a thinning agent comprised of propylene glycol.

Preferred applications of the method of the invention include use in the production of shampoo compositions and other anionic cleansing products. Advantageously, the method of the invention overcomes the problem of very high or irreversibly low viscosities of certain anionic compositions by providing a viscosity modifier which increases the salt tolerance level of these compositions without reducing peak viscosity.

Other objects, features and advantages of the present invention will be apparent when the detailed description of the preferred embodiments of the invention are considered in conjunction with the drawings, which should be construed in an illustrative and not limiting sense as follows:

Brief Description of the Drawings

FIG. 1 is a graphic illustration of a viscosity/salt curve for a SLES/CDEA composition;

FIG. 2 is a graphic illustration of a viscosity/salt curve for a ALS/betaine composition;

FIG. 3 is a graphic illustration of a viscosity/salt curve for a ALS/betaine composition containing Perfume 2;

FIG. 4 is a graphic illustration of a viscosity/salt curve for a ALS/betaine composition containing Perfume 1;

FIG. 5 is a graphic illustration of a viscosity/salt curve for a ALS/betaine composition containing Perfume 1 with the addition of SDS;

FIG. 6 is a graphic illustration of a viscosity/salt curve for a ALS/betaine composition containing Perfume 1 with the addition of SDS, salt and Solulan 75;

FIG. 7 is a graphic illustration of a viscosity/salt curve for a ALS/betaine composition containing Perfume 1 with the addition of SDS, salt and propylene glycol;

FIG. 8 and 9 are graphic illustrations viscosity/salt curves for a ALS/betaine composition containing Perfume 1 with the addition of SDS, salt and Ucon Fluid;

FIG. 10 is a graphic illustration of a viscosity/salt curve for a SLS/betaine composition containing Perfume 1; and

FIG. 11 is a graphic illustration of a viscosity/salt curve for a SLES/betaine composition containing Perfume 1.

DETAILED DESCRIPTION OF THE PREFERRED EMBODIMENT

In accordance with the present invention a viscosity modifier comprised of alkyl sulfates of the formula $R-OSO_3M$, in which R is a straight or branched chain alkyl of from 7 to 10 carbons and M is an alkali metal, is provided to adjust the viscosity of anionic surfactant compositions. In particular, sodium n-decyl sulfate (SDS) is used in the invention as the preferred alkyl sulfate. The viscosity modifier is used to increase the salt tolerance level of the anionic surfactant compositions without reducing peak viscosity. Preferably the anionic surfactant compositions comprise ammonium or sodium alkyl sulfates or alkyl ether sulfates.

The method for adjusting the viscosity of anionic surfactant compositions is accomplished by combining with the viscosity modifier to form a composition having a higher salt tolerance level. The viscosity modifier is present in the range of 0.05 to 5% of the composition and the anionic surfactant is present in the range of 8 to 25% of the composition.

An agent may be added to the resultant composition to adjust the composition to a desired viscosity. The agent may be a thickening agent comprised of salt or a thinning agent comprised of propylene glycol.

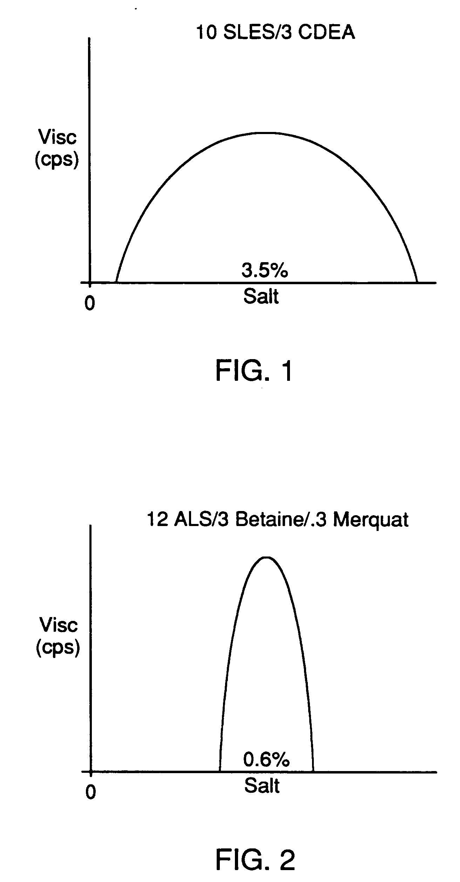

As shown in Figures 1 to 11, viscosity/salt curves are presented to demonstrate the salt tolerance levels of various anionic compositions and associated viscosity problems. Alkyl sulfates, particularly SDS, are effective for adjusting anionic surfactant compositions containing moderate to high active ingredient ammonium lauryl sulfate (ALS) which compositions have low salt tolerance levels. Addition of SDS to such compositions uniquely shifts the salt curve to the right, increasing salt tolerance of the compositions, eventually positioning the salt tolerance level low on the left side of the viscosity/salt curve where salt is normally added to adjust the compositions to a desired viscosity.

SLES and ALS Viscosity/Salt Curves

Anionic surfactant compositions such as sodium laureth (2EO) sulfate (SLES)/cocodiethanolamide (CDEA) provide a wide viscosity/salt curve that is only moderately salt sensitive and is about 3.5% salt tolerant, Figure 1. Ammonium lauryl sulfate (ALS) compositions, however, have a higher narrower viscosity/salt curve with a significantly lower salt tolerance of about 0.6%, Figure 2.

As illustrated in Figures 1 and 2, the addition of salt from the lower left side of the curves thickens the compositions to a maximum viscosity level. Beyond the peak, composition viscosity drops as excess salt is added. The ideal shampoo composition is one initially positioned on the low left side of the curve with lower than desired viscosity so it can be thickened with salt to within specification. This however is not always possible since the type and amount of active ingredients (AI) and % salt variations in raw materials have a direct influence on a composition's viscosity and its position on the salt curve.

Use of anionic compositions, particularly with ALS containing compositions at high active ingredient levels and those containing amphoterics such as betaines, develop very high nonpourable viscosities that generally require thinning agents to lower viscosity. In addition, surfactants from different manufacturers and perfumes contribute to viscosity problems of anionic surfactant compositions.

Free salts present in anionic and amphoteric raw materials also contribute to higher composition viscosities. The type of salt contained in surfactant compositions also has a significant effect on the viscosity/salt curve. In ALS compositions less ammonium chloride is required to thicken the composition as compared to compositions containing betaine in which higher amounts of sodium chloride are necessary to thicken the compositions, even though compositions containing betaine are usually higher in salt content. Therefore, ammonium salts in ALS compositions have a direct effect on the narrow ALS composition viscosity/salt curves. For example: If 42.1% (28% AI) ALS is used for a 12% ALS composition, then the following examples of contributory salt levels apply.

| % Salt in ALS Raw Material | = | % Salt in Shampoo |
|---|---|---|
| in spec (0.5 max) 0.476 | | 0.2 |
| over spec 0.714 | | 0.3 |

Consequently, minimal salt levels are desirable in ALS and betaine raw materials to assure that peak viscosity is not exceeded but is preferably positioned low on the left side of the viscosity/salt curves.

The following data and examples illustrate the method of the invention to adjust the viscosity of anionic surfactant compositions, particularly ALS compositions, with use of a viscosity modifier comprised of alkyl sulfates, preferably SDS, and other viscosity adjusting agents.

More specifically, Example I illustrates the problem of low irreversible viscosity levels in ALS shampoo compositions; Example II illustrates the addition of SDS as a viscosity modifier in ALS shampoo compositions; Examples III A, B and C show the effect of the addition of various thinning agents to ALS shampoo compositions containing SDS as a viscosity modifier; Example IV presents foam test data comparing the ALS shampoo compositions of Example I with an ALS shampoo composition containing SDS as a viscosity modifier; and Examples V and VI, respectively, illustrate the use of SDS as a viscosity modifier in other anionic surfactant compositions such as sodium lauryl sulfate (SLS) and sodium laureth sulfate (SLES).

EXAMPLE I

ALS SHAMPOO COMPOSITIONS

Conditioning shampoo compositions were prepared using an anionic surfactant composition comprised of ALS from two different manufacturers combined with two different perfumes, Perfume 1 and Perfume 2. Other ingredients in the compositions include betaine, a cationic conditioner, citric acid, colors, a preservative and salt. The particular cationic conditioner used in the compositions is dimethyldiallylammonium chloride/acrylamide copolymer, MERQUAT 550, having a molecular weight above 500,000, manufactured by Merck.

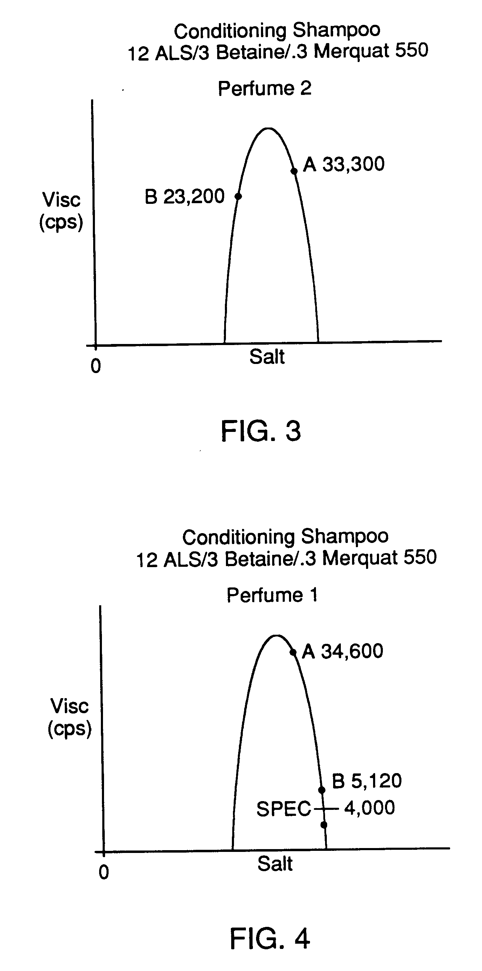

Viscosity/salt profiles of the compositions were determined by salt addition and are shown in Figures 3 and 4. Shampoo formulations A and B as shown in Table I below were comprised of 12 ALS/3 betaine/0.3 Merquat with either 0.8% of Perfume 1 or 0.6% of Perfume 2.

Shampoo A formulation included ALS (28% AI) - Standapol A, manufactured by Henkel Corporation, 300 Brookside Avenue, Amber, PA, 19002 and Shampoo B formulation included ALS (28% AI) - Eupicol AL-3, manufactured by Albright & Wilson Malaysia, PTE Ltd., No.5 Jalau Besut, Jurong Industrial Estate, Singapore, 2261.

TABLE I

EXAMPLE I CONDITIONING SHAMPOOS
12 ALS/3 Betaine/0.3 Merquat

|  | Shampoo A | | Shampoo B | |
| --- | --- | --- | --- | --- |
| Perfume: | 1 | 2 | 1 | 2 |
| Deionized Water | 41.495 | 41.695 | 41.495 | 41.695 |
| Citric Acid (50% Soln) | 0.120 | 0.120 | 0.120 | 0.120 |
| Cocamidopropylbetaine (30%) | 10.000 | 10.000 | 10.000 | 10.000 |
| Cationic Conditioner - Merquat 550 (8%) | 3.750 | 3.750 | 3.750 | 3.750 |
| Ammonium Lauryl Sulfate (28%) | 42.105 | 42.105 | 42.105 | 42.105 |
| Colors (Solns) | 0.180 | 0.180 | 0.180 | 0.180 |
| Perfume 1 | 0.800 | -- | 0.800 | -- |
| Perfume 2 | -- | 0.600 | -- | 0.600 |
| EDTA 62% Na₄ Dihydrate | 0.500 | 0.500 | 0.500 | 0.500 |
| Preservative - Kathon CG | 0.050 | 0.050 | 0.050 | 0.050 |
| Salt Allowance (±) not added | 1.000 | 1.000 | 1.000 | 1.000 |

As shown in Figures 3 and 4, both Shampoos A and B with Perfume 2 produced similar very high viscosities, but on opposite sides of the peak.

No viscosity problems were found using ALS (28% AI) - Standapol A, manufactured by Henkel with either Perfume 1 or 2. Shampoo A, in Figures 3 and 4, is positioned high on the right side for both Perfumes 1 and 2, indicating equal perfume response even in the presence of an excess salt situation.

The Shampoo B formula with Perfume 2 produced an acceptable viscosity level with the use of thinners such as propylene glycol, Ucon fluid 50HB660 or Solulan 75. However, Shampoo B is on the left side for Perfume 2, Figure 3, and low on the right side for Perfume 1, Figure 4.

5

Of the four formulations prepared in Table I, Shampoo B with Perfume 1 was the only one which showed a low irreversible viscosity level. The anionic surfactant used in the composition was ALS (28% AI) - Eupicol AL-3, manufactured by Albright & Wilson Malaysia. As previously stated, Figure 4, illustrates that Shampoo B formulation with Perfume 1 is positioned low on the right side of the curve. It is, however, important to be on the left side of the curve where final viscosity adjustments can be made with the use of salt, thinning agents, or a combination of both. Conversely, it is risky to be on the right side where viscosity is irreversible should excess salt or thinner be used or if the initial viscosity falls below specification limits.

The analytical comparison of Shampoos A and B, particularizing the ALS composition in each is found in Table II below.

TABLE II

| ALS ANALYTICAL COMPARISON OF EXAMPLE I COMPOSITIONS | | |
|---|---|---|
| | Shampoo A | Shampoo B |
| % Active ALS | 27.9 | 27.1 |
| % Free Oil | 1.0 | 1.5 |
| % $NH_4SO_4$ | 0.23 | 0.34 |
| % $NH_4Cl$ | 0.18 | 0.015 |
| % Water | 65.7 | 65.6 |

EXAMPLE II

ALS SHAMPOO COMPOSITIONS CONTAINING SDS

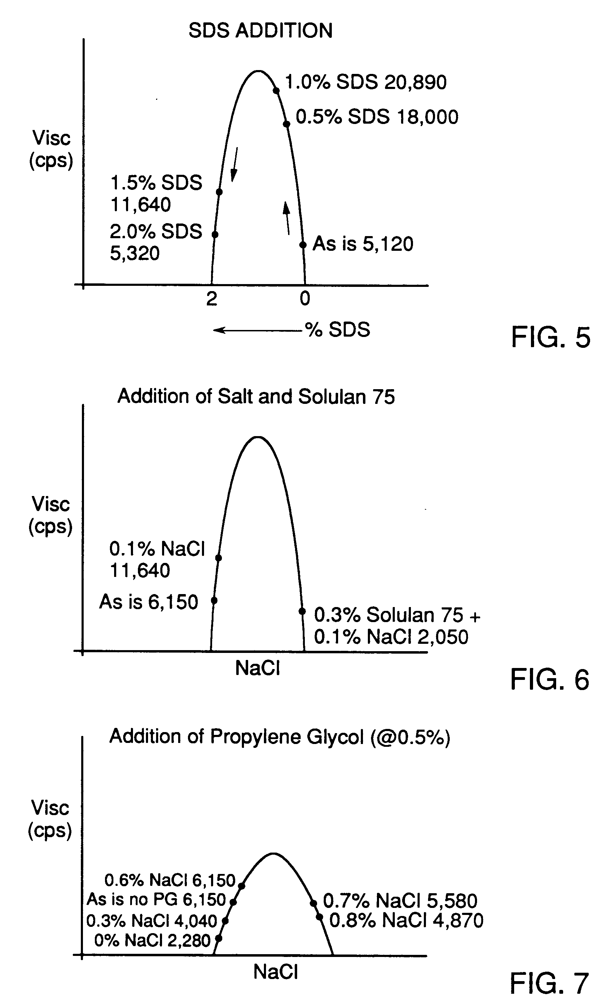

The effects on viscosity of the addition of sodium n-decyl sulfate (SDS) to ALS shampoo compositions were studied. SDS - Sulfotex 110, manufactured by Henkel Corporation was added in 0.5% AI increments to Shampoo B formulation with Perfume 1 from Example I. The results in Figure 5 show that the addition of SDS reversed the viscosity position of the shampoo composition by shifting the salt curve from right to left without noticeable reduction in the salt curve peak. The addition of SDS also functions as a foam booster.

Commonly used additives such as Na or $NH_4$ xylene sulfonates do not significantly modify the viscosities of an anionic surfactant composition as observed in Table III below. Xylene sulfonates were added in 0.5% AI increments to Shampoo B formulation with Perfume 1 from Example I.

TABLE III

| ADDITION OF Na or $NH_4$ XYLENE SULFONATE TO SHAMPOO B FORMULATION WITH PERFUME 1 | | | |
|---|---|---|---|
| Na Xylene Sulfonate | | $NH_4$ Xylene Sulfonate | |
| Addition (AI) | Viscosity(cps) | Addition (AI) | Viscosity(cps) |
| 0% | 1,900 | 0% | 1,900 |
| 0.5% | 1,100 | 0.5% | 1,100 |
| 1.0% | 1,100 | 1.0% | 2,110 |
| 1.5% | 3,840 | 1.5% | 4,650 |
| 2.0% | 4,890 | 2.0% | 4,090 |
| 2.5% | 2,840 | 2.5% | 2,020 |

EXAMPLE III

MODIFIED ALS COMPOSITIONS CONTAINING SDS

The following ALS/betaine composition was prepared to demonstrate the application of SDS in adjusting ALS composition viscosities and to examine the addition of common thinning agents. Examples A, B and C below illustrate the effect of the addition of the following thinning agents respectively, Solulan 75 manufactured by Amerchol Corp., 75 Eisenhower Parkway, Roseland, New Jersey, 07068-1691, propylene glycol and Ucon Fluid 50HB660, manufactured by Union Carbide, 136 Talmadge Road, P.O. Box 4051, Edison, New Jersey, 08818-4051, to the composition listed below.

**Modified Conditioning Shampoo Composition with 1.5% SDS**

(10.5 ALS/1.5 SDS/3 Betaine/0.3 Merquat 550)

| | |
|---|---|
| Deionized Water | 41.30 |
| Citric Acid (50% Soln) | 0.12 |
| Cocamidopropyl Betaine (30%) | 10.00 |
| Cationic Conditioner – Merquat 550 (8%) | 3.75 |
| Ammonium Lauryl Sulfate (same as Shampoo B formulation) | 37.50 |
| Sodium n-Decyl Sulfate (31%) | 4.80 |
| Colors | 0.18 |
| Perfume 1 | 0.80 |
| EDTA 62% Na$_4$ Dihydrate | 0.50 |
| Preservative – Kathon CG | 0.05 |
| Salt Allowance ± (not added) | 1.00 |
| | ------ |
| | 100.00 |

EXAMPLE III A

ADDITION OF SOLULAN 75 (ETHOXYLATED LANOLIN @ 0.3%)

As shown in Figure 6, the initial composition viscosity of 6,150 cps increased to 11,060 cps with only 0.1% salt addition. However, when 0.3% Solulan 75 was added to the composition, the viscosity dropped to 2,050 cps into an irreversible position on the right side of the salt curve. Solulan 75 was thus found to be an unsuitable thinning agent in this situation.

EXAMPLE III B

ADDITION OF PROPYLENE GLYCOL (@ 0.5%)

As shown in Figure 7, the initial viscosity of 6,150 cps was reduced to 2,280 cps by the addition of 0.5% propylene glycol then subsequently raised to within specification by the addition of 0.3% sodium chloride. Propylene glycol did not shift the salt curve as observed with Solulan in Example III A, however it did suppress the viscosity peak to about 6,300 cps in this instance. The addition of 0.5% propylene glycol thinned the shampoo sufficiently to permit final viscosity adjustment with salt addition. If a higher amount of

7

SDS were used, viscosity could have been reduced further to 4,000 cps or lower, thus making use of a thinning agent unnecessary.

EXAMPLE III C

ADDITION OF UCON FLUID 50HB660 @ 0.1%

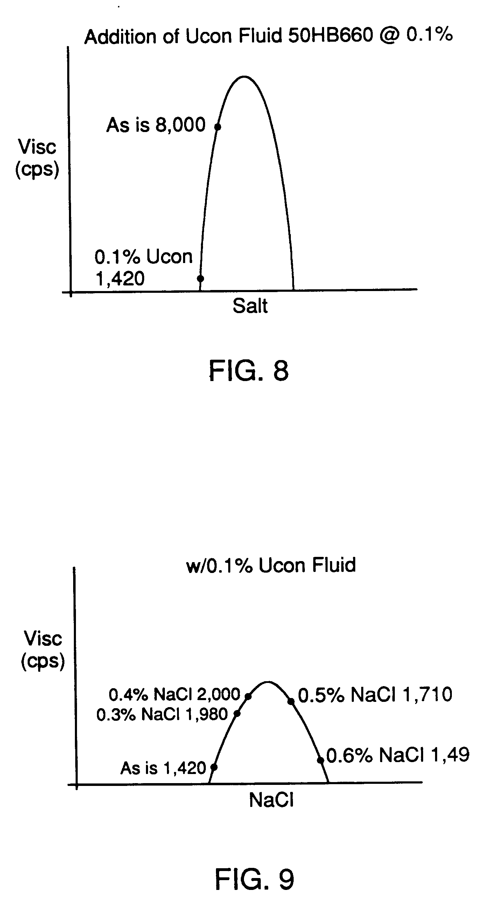

Another shampoo composition was prepared as in Examples III A and B above but had an initial viscosity of 8,000 cps. Figure 8 shows a drastic reduction in viscosity with just a 0.1% addition of Ucon fluid. Figure 9 illustrates an attempt to increase the viscosity of the composition above 2,000 cps with the addition of salt. In this case, however, the Ucon fluid lowered the viscosity peak below the desired viscosity of 4000 ± 500 cps, and was irreversible with the addition of salt.

Examples III A, B and C demonstrate the importance of knowing a composition's salt tolerence level and its position on the viscosity/salt curve to effectively overcome problems associated with selecting and using thinning agents to produce compositions with desired viscosities.

EXAMPLE IV

FOAM PROPERTIES OF ALS COMPOSITIONS CONTAINING SDS

Foam tests were performed on Shampoo A and B compositions from Example I (12 ALS/3 betaine/0.3 Merquat) and a mixed 10 ALS/2 SDS formula listed below in Table V. This latter composition was prepared using the same ALS composition as in Shampoo B and had an initial viscosity of 3,500 cps which is ideally located on the lower left side of the viscosity/salt curve. Therefore, no thinning agent was required and a final viscosity adjustment was made by minimal salt addition.

TABLE V

### Modified Conditioning Shampoo

(10 ALS/2 SDS/3 Betaine/0.3 Merquat 550)

| | |
|---|---|
| Deionized Water | 41.23 |
| Citric Acid (50% Soln) | 0.12 |
| Cocamidopropyl Betaine (30%) | 10.00 |
| Cationic Conditioner – Merquat 550 (8%) | 3.75 |
| Ammonium Lauryl Sulfate (28%) (same as Shampoo B formulation) | 35.71 |
| Sodium n-Decyl Sulfate (31%) | 6.66 |
| Colors | 0.18 |
| Perfume 1 | 0.80 |
| EDTA 62% Na$_4$ Dihydrate | 0.50 |
| Preservative – Kathon CG (1.5% Soln) | 0.05 |
| Salt Allowance ± (not added) | 1.00 |
| | ------ |
| | 100.00 |

## Foam Test Description

Laboratory Form Tests were performed on the compositions by inverting a 500 ml stoppered graduated cylinder 180° and returning it upright for 8 cycles (total 20) using an electric motor powered apparatus specifically designed for this purpose. Two determinations were taken: 8 cycles for flash foam and 12 additional cycles (total 20) for maximum foam properties. Flash foam tests determine the ease and speed with which a shampoo generates foam. Foam measurements after 20 cycles indicate the full lathering potential of a shampoo.

The following solution was made and heated to 105-108°F with mixing to melt and dissolve the synthetic sebum used as soil.

|  | gms |
|---|---|
| Shampoo | 15 |
| 150 ppm Hard Water | 85 |
| Synthetic Sebum | 3 |
|  | 103 gms |

Foam height in ml is measured along with drainage time in seconds. The latter is the time required for most of the water (100 ml) to drain out of the foam, and high values denote better foam stability often associated with foam creaminess.

## Foam Test Data

Foam test data shown in Table VI below are identical for Shampoo compositions A and B, and illustrate that a 2% SDS substitution for ALS did not adversely affect the foaming properties of the shampoo compositions.

### TABLE VI

| FOAM TEST DATA | | |
|---|---|---|
|  | Flash (8 cycles) | Maximum (20 cycles) |
| Shampoo A 12% ALS/Perfume 1 | 300 | 420/34 |
| Shampoo B 12% ALS/Perfume 1 | 300 | 420/24 |
| Shampoo B 10% ALS/2% SDS Modified formula | 305 | 410/27 |

## Adjusting Viscosity of Other Anionic Compositions with SDS

Sodium n-decyl sulfate was also found to adjust viscosities of other anionic surfactant compositions such as sodium lauryl, in Example V, or laureth sulfates, in Example IV, shown below. High viscosity was not a problem with these surfactant systems, so salt addition was necessary to create an excess salt situation in order to evaluate the performance of SDS in reversing the salt response.

## EXAMPLE V

## SODIUM LAURYL SULFATE (SLS) COMPOSITIONS CONTAINING SDS

The following composition was made using the same standard procedure as in Shampoos A and B from Example I but substituting SLS for ALS.

<u>SLS Shampoo Composition</u>

<u>12 SLS/3 Betaine/0.3 Merquat 550</u>

| | |
|---|---|
| Deionized Water | 43.00 |
| Citric Acid (50% Soln) | 0.12 |
| Cocamidopropyl Betaine (30%) | 10.00 |
| Cationic Conditioner - Merquat 550 (8%) | 3.75 |
| Sodium Lauryl Sulfate (29.5%) | 40.60 |
| Colors | 0.18 |
| Perfume 1 | 0.80 |
| EDTA 62% Na$_4$ Dihydrate | 0.50 |
| Preservative - Kathon CG | 0.05 |
| Salt Allowance ± | 1.00 |
| | ------ |
| | 100.00 |

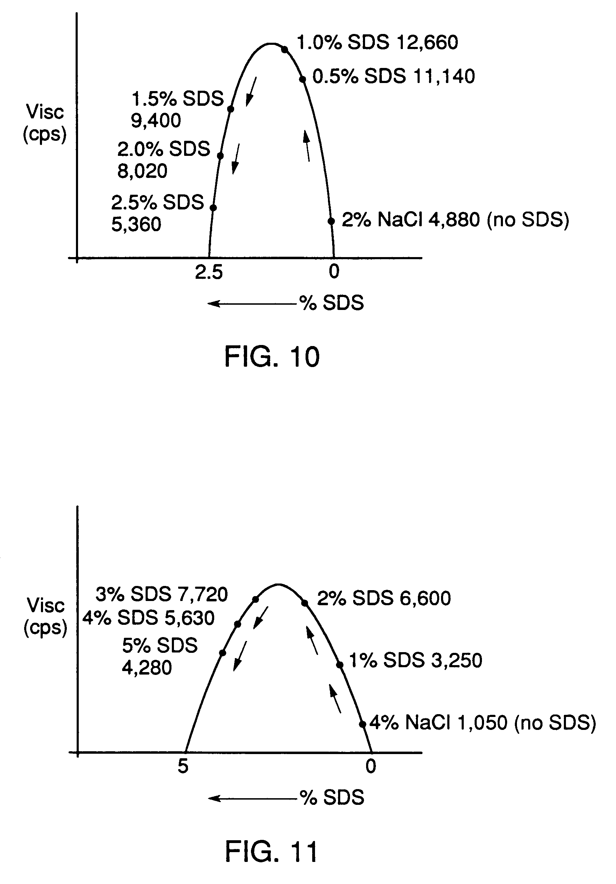

Initial viscosity of this composition without salt was 1,600 cps having an ideal position on the lower left side of the viscosity/salt curve (not illustrated). Addition of 2% salt brought the viscosity to 4,800 cps on the right side. As shown in Figure 10, subsequent addition of SDS in 0.5% increments produced a reversal typical of that observed with ALS shampoo.

EXAMPLE V

SODIUM LAURETH (2EO) SULFATE (SLES) COMPOSITIONS CONTAINING SDS

The following composition was made using the same standard procedure as in Shampoos A and B from Example I but substituting SLES for ALS.

## SLES Shampoo Composition

### 12 SLS/3 Betaine/0.3 Merquat 550

| | |
|---|---|
| Deionized Water | 36.54 |
| Citric Acid (50% Soln) | 0.12 |
| Cocamidopropyl Betaine (30%) | 10.00 |
| Cationic Conditioner - Merquat 550 (8%) | 3.75 |
| Sodium Lauryl (2EO) Sulfate (25.5%) | 47.00 |
| Colors | 0.18 |
| Perfume 1 | 0.80 |
| EDTA 62% Na$_4$ Dihydrate | 0.50 |
| Preservative - Kathon CG | 0.05 |
| Salt Allowance ± | 1.00 |
| | ------ |
| | 100.00 |

Initial viscosity of this composition without salt was 500 cps, positioned on the left side of the curve as shown in the Table VII below.

TABLE VII

| % NaCl | None | 1% | 2% | 3% | 4% |
|---|---|---|---|---|---|
| Viscosity (cps) | 500 | 17,460 | 38,000 | 15,400 | 1,050 |

As shown in Figure 11, subsequent addition of SDS in 1.0% increments also produced a reversal of the curve, but peak viscosity was reduced in this case to about 7,800 cps.

From the data and examples set forth above and related viscosity/salt curves it has been demonstrated that SLS compositions are generally more salt tolerant than ALS compositions. Example I showed that compositions made with different samples of ALS from different manufacturers may vary drastically in their viscosity/salt curve positions depending on the type of perfumes employed in the compositions.

Example III illustrates that the use of thinning agents such as Solulan 75, propylene glycol and Ucon fluid 50HB660 reduces the viscosity of anionic shampoo compositions but also adversely reduces peak viscosity of the resultant compositions. In particular, Solulan 75 (at 0.3% addition) adversely shifted the viscosity/salt curve to the left and was found to be unsuitable for shampoo compositions. Propylene glycol was found to have a milder thinning/peak viscosity reduction effect on anionic shampoo compositions.

Partial substitution of SDS for ALS in Example II increased the salt tolerance of the anionic compositions by uniquely shifting the viscosity/salt curve to the right, eventually reducing shampoo composition viscosity to an ideal position low on the left side of the salt curve. SDS substitution does not adversely affect peak viscosity like other thinning agents. As shown in Example IV, a 2% SDS substituted ALS shampoo composition foams comparably to a straight ALS shampoo composition of equal AI. Examples V and VI further illustrate that SDS is also effective as a viscosity modifier for SLS and SLES and other anionic surfactant systems.

It will be recognized by those skilled in the art that the method of the invention has wide applications which include use in the production of shampoo compositions and other anionic cleansing products. Advantageously, the method of the invention overcomes the problem of very high or irreversibly low viscosities of certain anionic compositions by providing a viscosity modifier which increases the salt tolerance level of these compositions without reducing peak viscosity.

11

EP 0 574 086 A2

Numerous modifications are possible in light of the above disclosure incorporating the use of other anionic surfactant systems, low salt raw materials, and partial substitution of SDS for ALS or other anionic surfactant compositions as an alternate to thinning agents.

Therefore, although the invention has been described with respect to illustrations and examples thereof it is not to be limited to those because it is considered that one of skill in the art will be able to utilize substitutes and equivalents to make such compositions without departing from the scope and spirit of the invention as defined in the claims appended hereto.

**Claims**

1. A viscosity modifier of anionic surfactant compositions which comprises an alkyl sulfate of the formula:

$$R-OSO_3M$$

in which R is a straight or branched chain alkyl of from 7 to 10 carbons and M is an alkali metal.

2. The viscosity modifier according to claim 1, wherein said alkyl sulfate is sodium n-decyl sulfate.

3. The viscosity modifier according to claim 1, wherein said anionic surfactant is selected from the group comprising ammonium or sodium alkyl or alkyl ether sulfates.

4. The viscosity modifier according to claim 1, wherein said anionic surfactant is ammonium lauryl sulfate.

5. The viscosity modifier according to claim 1, wherein said alkyl sulfate is present in the range 0.05 to 5% of said composition.

6. A method for adjusting the viscosity of anionic surfactant compositions comprising the steps of:
   providing an anionic surfactant composition;
   combining said anionic surfactant composition with a viscosity modifier which comprises an alkyl sulfate of the formula:

$$R-OSO_3M$$

   in which R is a straight or branched chain alkyl of from 7 to 10 carbons and M is an alkali metal to form a composition having a higher salt tolerance level; and
   adding an agent to adjust said composition to desired viscosity.

7. The method according to claim 6, wherein said alkyl sulfate is sodium n-decyl sulfate.

8. The method according to claim 6, wherein said anionic surfactant is selected from the group comprising ammonium or sodium alkyl or alkyl ether sulfates.

9. The method according to claim 6, wherein said anionic surfactant is ammonium lauryl sulfate.

10. The method according to claim 6, wherein said alkyl sulfate is present in the range 0.05 to 5% of said composition.

11. The method according to claim 6, wherein said anionic surfactant is present in the range of 8 to 25% of said composition.

12. The method according to claim 6, wherein said agent is a thickening agent comprised of salt.

13. The method according to claim 6, wherein said agent is a thinning agent comprised of propylene glycol.

12

10 SLES/3 CDEA

Visc (cps)

3.5% Salt

0

FIG. 1

12 ALS/3 Betaine/.3 Merquat

Visc (cps)

0.6% Salt

0

FIG. 2

Conditioning Shampoo
12 ALS/3 Betaine/.3 Merquat 550

Perfume 2

Visc (cps)

A 33,300

B 23,200

Salt

0

## FIG. 3

Conditioning Shampoo
12 ALS/3 Betaine/.3 Merquat 550

Perfume 1

Visc (cps)

A 34,600

B 5,120

SPEC — 4,000

Salt

0

## FIG. 4

## SDS ADDITION

1.0% SDS 20,890

0.5% SDS 18,000

1.5% SDS
11,640

2.0% SDS
5,320

As is 5,120

2　　0

←――→ % SDS

**FIG. 5**

## Addition of Salt and Solulan 75

0.1% NaCl
11,640

As is 6,150

0.3% Solulan 75 +
0.1% NaCl 2,050

NaCl

**FIG. 6**

## Addition of Propylene Glycol (@0.5%)

0.6% NaCl 6,150
As is no PG 6,150
0.3% NaCl 4,040
0% NaCl 2,280

0.7% NaCl 5,580
0.8% NaCl 4,870

NaCl

**FIG. 7**

Addition of Ucon Fluid 50HB660 @ 0.1%

Visc
(cps)

As is 8,000 •

0.1% Ucon
1,420 •

Salt

## FIG. 8

w/0.1% Ucon Fluid

Visc
(cps)

0.4% NaCl 2,000 • ⟍ •0.5% NaCl 1,710
0.3% NaCl 1,980 •

•0.6% NaCl 1,49

As is 1,420 •

NaCl

## FIG. 9

16

FIG. 10

FIG. 11